(19) Europäisches Patentamt
European Patent Office
Office européen des brevets

(11) Numéro de publication: **0 200 621 B1**

# (12) FASCICULE DE BREVET EUROPEEN

(45) Date de publication du fascicule du brevet: **09.03.88**

(51) Int. Cl.⁴: **C 07 C 29/14**, C 07 C 31/40

(21) Numéro de dépôt: **86400802.4**

(22) Date de dépôt: **15.04.86**

(54) **Procédé de synthèse du trifluoro-2,2,2 éthanol.**

(30) Priorité: **23.04.85 FR 8506121**

(43) Date de publication de la demande: **10.12.86 Bulletin 86/45**

(45) Mention de la délivrance du brevet: **09.03.88 Bulletin 88/10**

(84) Etats contractants désignés: **AT BE CH DE FR GB IT LI LU NL SE**

(56) Documents cités:
**EP - A - 0 149 905**
**GB - A - 2 109 791**

(73) Titulaire: **ATOCHEM, 4 & 8, Cours Michelet La Défense 10, F-92800 Puteaux (FR)**

(72) Inventeur: **Cheminal, Bernard Rés. Beauséjour Saint-Irénée, Lieu-dit La Rivière Orlienas, F-69530 Brignais (FR)**
Inventeur: **Mathais, Henri, 87, Chemin de l'Indiennerie, F-69370 Saint-Didier-au-Mont-d'Or (FR)**

(74) Mandataire: **Leboulenger, Jean et al, ATOCHEM Département Propriété Industrielle, F-92091 Paris la Défense 10 Cédex 42 (FR)**

EP 0 200 621 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la fabrication de trifluoro-2,2,2 éthanol par hydrogénolyse d'hydrates ou d'hémiacétals du trifluoro-2,2,2 acétaldéhyde (fluoral).

Une importante source industrielle d'alcools primaires fluorés comme le trifluoro-2,2,2 éthanol, qui est utile dans un large domaine d'applications telles que la récupération d'énergie (pompes à chaleur à absorption), les produits pharmaceutiques (anesthésiques) et les solvants, provient de la réduction de l'acide correspondant (acide trifluoroacétique en l'occurrence) ou d'un dérivé (ester, chlorure d'acide, anhydride, amide) par l'hydrogène en présence d'un catalyseur le plus souvent choisi dans la famille des métaux précieux (rhodium, rhuténium, platine, palladium). Parmi les principales techniques employées, on peut citer l'hydrogénation de l'anhydride trifluoroacétique (brevet U.S. 4255594), l'hydrogénation de l'acide trifluoroacétique (brevets U.S. 4273947, FR 2544712 et FR 2545481), l'hydrogénation des esters de l'acide trifluoroacétique (brevet EP 36939), l'hydrogénation du trifluoroacétamide [M. GILMAN, J.A.C.S., *70*, 1281-2 (1948)], l'hydrogénolyse du chlorure de trifluoroacétyle (brevet U.S. 3970710). Outre l'inconvénient d'une mauvaise tenue du catalyseur dans le temps, ces procédés présentent le désavantage économique de recourir à une oxydation des matières premières (la plupart du temps chlorées) pour accéder à l'acide ou à l'un de ses dérivés, suivie d'une réduction de cet acide en alcool; cette étape supplémentaire grève très sérieusement la rentabilité de ces procédés.

Une autre famille de procédés consiste à hydrogéner le fluoral ou l'un de ses dérivés. Le rendement obtenu par hydrogénation en phase liquide (80°C sous 95 bars) de l'hydrate de fluoral surcatalyseur au nickel (brevet FR 1399290) est médiocre: la durée de vie du catalyseur n'est pas mentionnée et la réaction requiert une grande quantité de catalyseur (16% en poids de nickel pur par rapport au fluoral) dans des conditions opératoires très sévères. On peut citer également le brevet U.S. 2982789 qui décrit l'hydrogénation en phase gazeuse du chlorhydrate de fluoral: $CF_3CH(Cl)OH$ provenant d'une première étape d'hydrogénolyse (réaction de ROSENMUND) du chlorure de trifluoroacétyle sur catalyseur au palladium; le catalyseur d'hydrogénation du fluoral, qui consiste en chromite de cuivre déposé sur fluorure de calcium et dont la tenue dans le temps n'est pas mentionnée, fonctionne vers 250°C et ne permet de transformer le fluoral intermédiaire que d'une manière incomplète (environ 60-65%); en outre, le recyclage du chlorhydrate de fluoral non transformé est une opération très risquée à cause de son instabilité thermique, la décomposition:

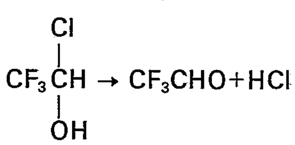

(1)

étant favorisée dès 30°C par une élévation de la température ou une diminution de la pression. On peut citer enfin l'hydrogénation du fluoral en phase gazeuse (brevet U.S. 3468964) en présence d'un catalyseur au palladium déposé sur alumine à basse température (température de pointe: 140°C); le rendement modeste en trifluoroéthanol (86%) et l'obligation de régénérer très souvent le catalyseur à 200°C dans l'oxygène pur, ainsi que l'extrême difficulté de véhiculer le fluoral à l'état pur (polymérisations), rendent le procédé non attractif.

La présente invention permet de remédier à tous ces inconvénients en proposant une technique simple, souple et particulièrement économique pour accéder au trifluoro-2,2,2 éthanol, et cela même à partir d'une matière première brute facile à préparer et à transporter sans nuire ni à la durée de vie du catalyseur, ni au rendement, ni à la productivité.

Le procédé selon l'invention consiste à soumettre un composé de formule générale:

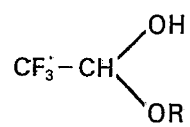

(I)

dans laquelle R représente un atome d'hydrogène ou un radical alkyle linéaire ou ramifié, comprenant de 1 à 8 atomes de carbone et éventuellement partiellement fluoré, à une hydrogénolyse en phase liquide en présence d'un catalyseur au nickel et d'un cocatalyseur choisi parmi les amines tertiaires aliphatiques.

Les composés de formule (I), utilisés comme matières premières dans le procédé selon l'invention, peuvent être obtenus, à l'état brut, de manière connue par action de l'eau (R = H) ou d'un alcool (R = alkyl éventuellement substitué) sur le fluoral selon le schéma réactionnel suivant:

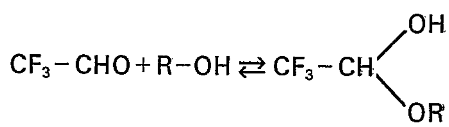

(2)

Comme exemples de radicaux alkyle R, on peut citer plus particulièrement les radicaux méthyle, éthyle, n. propyle, isopropyle, n. butyle, sec.-butyle, n. hexyle, éthyl-2 hexyle, trifluoro-2,2,2 éthyle et hexafluoro-1,1,1,3,3,3 propyl-2.

La stabilité thermique des composés obtenus dépend essentiellement de la température, de la pression et de leur nature chimique. D'une façon générale, les hydrates (R = H) sont stables mais difficiles à purifier par distillation. Les hémiacétals (R = alkyl) sont d'autant plus stables que le nombre d'atomes de carbone de R est faible; leur purification exige parfois une distillation sous léger vide (200 torr) et un temps de séjour court (évaporation en film tombant). A titre d'exemples, l'hémiacétal $CF_3-CH(OH)-OCH_3$ brut est stable et peut être distillé (point d'ébullition: 96°C/760 torr) à la pression atmosphérique sans décomposition,

donc séparé des composés chlorés (plus lourds) et de l'acide chlorhydrique; on obtient alors un produit titrant $\geqslant$ 99,9% lorsque l'on évite un temps de séjour trop long (environ quelques minutes) dans le bouilleur de l'appareil à distiller. Par contre, l'hémiacétal $CF_3-CH(OH)-OCH_2CF_3$, très instable, ne peut pas être purifié par distillation; on peut néanmoins, conformément à la présente invention, l'utiliser tel quel à l'état brut, c'est-à-dire sous forme de mélange comprenant de 1 à 10 moles pour cent d'hémiacétal chloré $CF_2-Cl-CH(OH)-COH_2CF_3$, de 0,1 à 10 moles pour cent d'hémiacétal dichloré $CFCl_2-CH(OH)-OCH_2CF_3$ et de 1 à 10 moles pour cent d'acide chlorhydrique.

La teneur en nickel du catalyseur utilisé dans le procédé selon l'invention peut varier entre 30 et 90% en poids, mais on emploie de préférence un catalyseur à environ 60% de nickel. La quantité de catalyseur à mettre en œuvre dépend évidemment de sa teneur en nickel; pour un catalyseur commercial à 64% de nickel, cette quantité peut varier entre 0,2 et 7,5% et de préférence entre 0,5 et 2% par rapport au poids de substrat (hydrate ou hémiacétal) brut mis en œuvre.

Le catalyseur au nickel utilisé peut être, par exemple, du nickel de RANEY obtenu par attaque alcaline (notamment sodique) d'un alliage 50/50 de nickel et d'aluminium, puis lavage à l'eau et éventuellement avec une solution aqueuse diluée d'acide acétique. De préférence, on utilise un catalyseur au nickel commercial déposé sur un support classique tel que la silice ou, de préférence, le kieselguhr. Ce genre de catalyseur est en général fourni sous une forme stabilisée (préréduit avec une légère oxydation en surface) de manière à faciliter son transport et sa manipulation et à le rendre ainsi non pyrophorique.

Le catalyseur peut être employé tel quel avec ou sans activation préalable dans le réacteur, avant l'opération d'hydrogénolyse; cette éventuelle activation peut être menée, par exemple, à une température comprise entre 140 et 200°C, de préférence entre 170 et 180°C, sous une pression d'hydrogène de 30 à 45 bars. Une telle activation est cependant nécessaire lorsqu'on emploie un catalyseur commercial stabilisé.

Après chaque opération d'hydrogénolyse, le catalyseur peut être utilement séparé du mélange réactionnel par décantation puis filtration suivie d'un ou plusieurs lavages à l'eau ou à l'alcool pur synthétisé dans le procédé; on peut également le laisser séjourner (de préférence moins de 48 heures) en contact avec le mélange réactionnel, le laisser décanter, puis séparer le mélange réactionnel par soutirage sous atmosphère d'hydrogène et recommencer une opération suivante sur la même charge catalytique. On peut également mettre à profit le caractère magnétique du catalyseur pour le séparer du mélange réactionnel.

Les réactifs (substrat + cocatalyseur) peuvent être introduits avec le catalyseur au début de la réaction. Cependant, pour éviter une désactivation trop rapide du catalyseur dans le temps, il est préférable d'introduire progressivement le mélange des réactifs dans un mélange constitué d'eau ou bien d'alcool brut issu de la synthèse précédente (ou d'alcool purifié) et de catalyseur.

L'hydrogénolyse peut être effectuée à une température comprise entre 150 et 200°C, de préférence entre 170 et 180°C, et sous une pression comprise entre environ 20 et 50 bars, de préférence entre 30 et 45 bars.

Dans le cas où, conformément à une modalité préférée du procédé selon l'invention, on introduit progressivement les réactifs (substrat + cocatalyseur) dans le réacteur d'hydrogénolyse, la durée d'introduction peut varier entre 0,2 et 5 heures, de préférence entre 0,75 et 1,25 heure. Afin d'éviter une désactivation trop rapide du catalyseur, il est particulièrement avantageux que la vitesse d'introduction des réactifs corresponde le plus exactement possible à la vitesse d'hydrogénolyse. Le système de régulation le plus approprié pour parvenir à ce résultat consiste à asservir le débit de la pompe d'injection à la consommation d'hydrogène (elle-même liée au débit ou à la pression d'hydrogène); au fur et à mesure de la lente désactivation du catalyseur dans le temps, on peut ainsi compenser sa légère perte d'activité par une augmentation correspondante de la durée d'introduction des réactifs; cela augmente considérablement la souplesse du procédé en même temps que son économie.

Comme cocatalysur, on utilise les amines tertiaires alphatiques de formule:

$$R_1-\underset{\displaystyle R_3}{\underset{|}{N}}-R_2 \qquad (II)$$

dans laquelle les symboles $R_1$, $R_2$ et $R_3$ peuvent être identiques ou différents et représentent chacun un radical alkyle éventuellement substitué par un groupe hydroxy. Bien que l'on préfère utiliser la diméthyléthylamine ou la tributylamine, on peut mentionner aussi, à titre d'exemples non limitatifs, la triméthylamine, la triéthylamine, la tri-n.propylamine, la diméthyl-éthanolamine, la triéthanolamine.

La quantité de cocatalyseur peut varier dans de larges limites en fonction de la pureté du substrat (hydrate ou hémiacétal) soumis à l'hydrogénolyse. Il est évidemment souhaitable de partir d'un substrat aussi pur que possible, mais cela n'est pas toujours réalisable (catalyseur de fluoruration moins performant conduisant à une teneur plus élevée en produits chlorés, réaction secondaire de l'alcool obligeant à absorber le dérivé carbonylé à basse température, ce qui accroît la solubilité de l'acide chlorhydrique, hydrates chlorés et fluorés inséparables par distillation, etc.). La quantité de cocatalyseur à employer est en général comprise entre environ 1,05 et 6 moles, de préférence entre 1,05 et 4 moles, par atome-gramme de chlore des sous-produits chlorés contenus dans la matière première brute.

Le solvant utilisé peut être l'eau ou n'importe quel solvant organique habituel (cétones aliphatiques, éthers, glycols, solvants chlorés), mais on utilise de préférence le trifluoro-2,2,2 éthanol fourni par le procédé selon l'invention; on peut

également employer un alcool aliphatique R−OH (R ayant la même signification que ci-dessus). La quantité de solvant à utiliser peut varier de 0 à 100% par rapport au poids du substrat mis en œuvre et ne dépend que de la géométrie du réacteur d'hydrogénolyse (en particulier pour assurer une excellente agitation) et de la productivité visée.

Le procédé selon l'invention peut être mis en œuvre dans un appareil de type classique, c'est-à-dire un autoclave agité par un moyen mécanique adéquat, capable de fonctionner sous une pression de 50 bars et muni d'un dispositif de soutirage de la suspension catalytique et des annexes indispensables (filtre, pompes, régulation de pression d'hydrogène, etc.). Le milieu basique garantissant contre la corrosion, le réacteur peut être réalisé simplement en acier inoxydable (NS 22 S).

L'hydrogénolyse selon l'invention peut également être menée en phase liquide continue sur un lit fixe de catalyseur.

Le trifluoro-2,2,2 éthanol produit peut être isolé et purifié par les méthodes conventionnelles telles que la distillation et le séchage sur tamis moléculaire. Sa pureté peut être déterminée par chromatographie en phase gazeuse.

Dans les exemples suivants qui illustrent l'invention sans la limiter, le catalyseur utilisé est un catalyseur standard, fourni par la Société HARSHAW sous la désignation Ni 5132 P; il contient 64% de nickel déposé sur kieselguhr et se présente sous la forme d'une poudre fine dont tous les grains ont un diamètre supérieur à 0,5 µm. Ce catalyseur étant rendu non pyrophorique par une légère oxydation en surface pour faciliter sa manipulation et son transport, une activation «in situ» par l'hydrogène est nécessaire avant le démarrage de tout essai.

*Exemple 1:*

Dans un autoclave de 0,1 litre muni d'un système d'agitation par barreau magnétique, on charge successivement 1,82 g de catalyseur Ni 5132 P, puis 21 g de trifluoro-2,2,2 éthanol. On ferme le réacteur, purge l'air emmagasiné avec de l'azote et introduit une légère pression d'hydrogène, puis on chauffe le mélange jusqu'à environ 175°C sous agitation et ajuste la pression d'hydrogène à 38 bars; l'activation du catalyseur dure 20 minutes dans ces conditions.

On introduit alors progressivement (environ 57 minutes) dans le réacteur un mélange de 2,1 g de diméthyléthylamine (0,0287 mole) et 31,3 g d'hémiacétal brut contenant (en moles) 95,5% de $CF_3-CH(OH)-O-CH_2-CF_3$ et 4,5% de $CF_2Cl-CH(OH)-O-CH_2-CF_3$, ce qui correspond respectivement à 0,151 mole et 0,007 mole, l'acide fluorhydrique dissous représentant $3{,}33 \cdot 10^{-5}$ mole.

La baisse de pression correspondant à la consommation d'hydrogène est ensuite compensée par des introductions successives d'hydrogène entre 35 et 45 bars. Une fois l'absorption d'hydrogène terminée (après 65 minutes), on refroidit rapidement le mélange réactionnel puis, après l'avoir dégazé dans ce récipient maintenu vers −196°C par de l'azote liquide, on procède à l'ouverture du réacteur et le produit de la réaction mélangé au catalyseur est transféré dans un récipient dans lequel on laisse décanter la suspension catalytique. Après séparation du catalyseur, le produit de l'hydrogénolyse est analysé sur un échantillon. On note le pH d'une solution aqueuse et l'on détermine par analyse minérale les ions chlorures ($Cl^-$) et fluorures ($F^-$) formés durant la réaction.

Le tableau rassemble les résultats obtenus dans cet exemple et dans les exemples 2 à 8 suivants.

*Exemple 2:*

On opère comme à l'exemple 1, mais en remplaçant l'hémiacétal par 32,4 g d'hydrate de fluoral brut ayant la composition suivante:

| | |
|---|---|
| $CF_3-CH(OH)_2$ | 0,244 mole |
| $CF_2Cl-CH(OH)_2$+HCl dissous | 0,0066 mole |
| HF dissous | $4{,}91 \cdot 10^{-5}$ mole |
| $H_2O$ | 0,189 mole |

Après 1 heure à 175°C sous environ 32 à 45 bars, la réaction est complète et le rendement en trifluoro-2,2,2 éthanol est quantitatif.

*Exemple 3:*

On répète l'exemple 2, mais en utilisant seulement 0,36 g de catalyseur. Après 110 minutes à 175°C sous 37 à 46 bars d'hydrogène, la réaction est complète et le rendement en trifluoro-2,2,2 éthanol est quantitatif.

*Exemple 4:*

On opère comme à l'exemple 1 avec 0,38 g de catalyseur, 0,32 g de diméthyléthylamine pure ($4{,}38 \cdot 10^{-3}$ mole) et 40,1 g d'hydrate de fluoral brut ayant la composition suivante:

| | |
|---|---|
| $CF_3-CH(OH)_2$ | 0,247 mole |
| $CF_2Cl-CH(OH)_2$ | 0,003 mole |
| HCl dissous | $1{,}7 \cdot 10^{-5}$ mole |
| HF dissous | $1{,}21 \cdot 10^{-4}$ mole |
| $H_2O$ | 0,619 mole |

Après 95 minutes à 175°C sous 27 à 46 bars d'hydrogène, la réaction est complète et le rendement en trifluoro-2,2,2 éthanol est quantitatif.

*Exemple 5:*

On répète l'exemple 4, mais en utilisant seulement 0,26 g de diméthyléthylamine ($3{,}56 \cdot 10^{-3}$ mole). Après 100 minutes à 175°C sous 29 à 46 bars d'hydrogène, la réaction est complète et le rendement en trifluoro-2,2,2 éthanol est quantitatif.

*Exemple 6:*

(a) On répète l'exemple 4, mais en remplaçant la diméthyléthylamine par 1,39 g de tributylamine ($7{,}5 \cdot 10^{-3}$ mole). La réaction est terminée après 80 minutes à 175°C sous 33 à 48 bars d'hydrogène.

(b) Le catalyseur provenant de l'opération (a) est réutilisé pour une nouvelle opération réalisée dans les mêmes conditions avec les mêmes quantités d'hydrate brut et de tributylamine. La réaction dure 95 minutes.

| Exemple | pH | $Cl^-$ créé | $F^-$ créé |
|---|---|---|---|
| | | (pour 100 moles de $C_2$*) | |
| 1 | 10,05 | 1,54 | 0,27 |
| 2 | 8,65 | 5,44 | 0,30 |
| 3 | 8,80 | 5,73 | 0,59 |
| 4 | 7,20 | 0,83 | 0,22 |
| 5 | 6,80 | 0,65 | 0,16 |
| 6(a) | 8,90 | 0,91 | 0,57 |
| 6(b) | 7,50 | 0,91 | 0,57 |

* $C_2$ désigne les composés $CF_xCl_{3-x}CHO$, x étant égal à 1, 2 ou 3.

*Exemple comparatif:*

On répète l'exemple 4, mais sans addition de diméthyléthylamine. Après 7 heures à 175°C sous 33 à 46 bars d'hydrogène, le taux de transformation du fluoral en trifluoro-éthanol n'est que de 40%. On trouve:

| | |
|---|---|
| pH | 5,1 |
| $Cl^-$ créé (pour 100 moles de $C_2$) | 0,6 |
| $F^-$ créé (pour 100 moles de $C_2$) | 0 |

## Revendications

1. Procédé de synthèse du trifluoro-2,2,2 éthanol par hydrogénolyse catalytique en phase liquide d'un composé de formule:

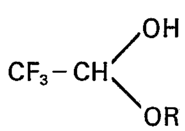

(I)

dans laquelle R est un atome d'hydrogène ou un radical alkyle, linéaire ou ramifié, comprenant de 1 à 8 atomes de carbone et éventuellement partiellement fluoré, en présence d'un catalyseur au nickel, caractérisé en ce que l'on opère en présence d'un cocatalyseur choisi parmi les amines tertiaires aliphatiquees.

2. Procédé selon la revendication 1, dans lequel on introduit progressivement un mélange d'un composé de formule (I) et d'un cocatalyseur dans une suspension du catalyseur dans l'eau ou dans le trifluoro-2,2,2 éthanol.

3. Procédé selon la revendication 1 ou 2, dans lequel, après cessation de la réaction, on refroidit rapidement le mélange réactionnel, sépare le catalyseur et le réutilise dans une opération ultérieure.

4. Procédé selon la revendication 3, dans lequel on sépare le catalyseur par filtration puis le lave à l'eau avant réutilisation.

5. Procédé selon la revendication 3, dans lequel, après refroidissement du mélange réactionnel, on laisse décanter le catalyseur, puis soutire le liquide réactionnel et réutilise tel quel le catalyseur maintenu dans le réacteur.

6. Procédé selon l'une des revendications 1 à 5, dans lequel on emploie un composé de formule (I) à l'état brut.

7. Procédé selon l'une des revendications 1 à 6, dans lequel on opère à une température allant de 150 à 200°C, de préférence entre 170 et 180°C.

8. Procédé selon l'une des revendications 1 à 7, dans lequel on opère sous une pression d'environ 20 à 50 bars, de préférence entre 30 et 45 bars.

9. Procédé selon l'une des revendications 1 à 8, dans lequel la teneur en nickel du catalyseur varie de 30 à 90% en poids et est, de préférence, égale à environ 64%.

10. Procédé selon l'une des revendications 1 à 9, dans lequel le catalyseur est préalablement activé à une température comprise etnre 140 et 200°C sous une pression d'hydrogène de 30 à 45 bars.

11. Procédé selon l'une des revendications 1 à 10, dans lequel la quantité de catalyseur, exprimée pour un catalyseur à 64% de nickel, est comprise entre 0,2 et 7,5%, de préférence entre 0,5 et 2%, par rapport au poids d'hydrate ou d'hémiacétal mis en œuvre.

12. Procédé selon l'une des revendications 1 à 11, dans lequel la quantité de cocatalyseur est comprise entre environ 1,05 et 6 moles, de préférence entre 1,05 et 4 moles, par atome-gramme de chlore des sous-produits chlorés contenus dans le composé de formule (I) brut.

13. Procédé selon la revendication 12, dans lequel le cocatalyseur est une amine de formule $R_1-N(R_2)R_3$ dans laquelle les symboles $R_1$, $R_2$, $R_3$, identiques ou différents, représentent chacun un radical alkyle éventuellement substitué par un groupe hydroxy.

## Patentansprüche

1. Verfahren zur Herstellung von 2,2,2-Trifluorethanol durch katalytische Hydrogenolyse einer Verbindung der nachfolgenden Formel in flüssiger Phase:

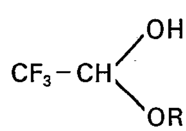

(I)

in der R ein Wasserstoffatom oder ein linearer oder verzweigter Alkylrest mit 1 bis 8 Kohlenstoffatomen ist, der gegebenenfalls partiell fluoriert ist, in Gegenwart eines Nickelkatalysators, dadurch gekennzeichnet, dass man in Gegenwart eines Cokatalysators ausgewählt aus den tertiären aliphatischen Aminen arbeitet.

2. Verfahren nach Anspruch 1, bei dem man nach und nach ein Gemisch einer Verbindung der Formel (I) und eines Cokatalysators zu einer Suspension des Katalysators in Wasser oder in 2,2,2-Trifluorethanol zugibt.

3. Verfahren nach Anspruch 1 oder 2, bei dem man nach Beendigung der Reaktion das Reaktionsgemisch schnell abkühlt, den Katalysator abtrennt und diesen in einem späteren Arbeitsschritt wieder verwendet.

4. Verfahren nach Anspruch 3, bei dem man den Katalysator durch Filtration abtrennt und diesen anschliessend vor der Wiederverwendung mit Wasser wäscht.

5. Verfahren nach Anspruch 3, bei dem man nach der Abkühlung des Reaktionsgemischs den Katalysator sich absetzen lässt, dann die Reaktionsflüssigkeit abzieht und den in Reaktor verbliebenen Katalysator so wieder verwendet.

6. Verfahren nach einem der Ansprüche 1 bis 5, bei dem man eine Verbindung der Formel (I) im Rohzustand verwendet.

7. Verfahren nach einem der Ansprüche 1 bis 6, bei dem man bei einer Temperatur zwischen 150 und 200°C, vorzugsweise zwischen 170 und 180°C arbeitet.

8. Verfahren nach einem der Ansprüche 1 bis 6, bei dem man bei einem Druck von ungefähr 20 bis 50 bar, vorzugsweise zwischen 30 und 45 bar arbeitet.

9. Verfahren nach einem der Ansprüche 1 bis 8, bei dem der Nickelgehalt des Katalysators zwischen 30 und 90 Gew.-% beträgt und vorzugsweise ungefähr gleich 64% ist.

10. Verfahren nach einem der Ansprüche 1 bis 9, bei dem der Katalysator vorher bei einer Temperatur zwischen 140 und 200°C bei einem Wasserstoffdruck zwischen 30 und 45 bar aktiviert wird.

11. Verfahren nach einem der Ansprüche 1 bis 10, bei dem die Menge des Katalysators, bezogen auf einen Katalysator mit 64% Nickelgehalt, zwischen 0,2 und 7,5%, vorzugsweise zwischen 0,5 und 2%, bezogen auf das Gewicht des umgesetzten Hydrats oder Hemiacetals beträgt.

12. Verfahren nach einem der Ansprüche 1 bis 11, bei dem die Menge des Cokatalysators zwischen ungefähr 1,05 und 6 Mol, vorzugsweise zwischen 1,05 und 4 Mol, pro Gramm-Atom Chlor der chlorierten Nebenprodukte, die in der Verbindung der Formel (I) im Rohzustand enthalten sind, beträgt.

13. Verfahren gemäss Anspruch 12, bei dem der Cokatalysator ein Amin der Formel $R_1-N(R_2)R_3$ ist, in der die Reste $R_1$, $R_2$, $R_3$ identisch oder unterschiedlich sind und jeweils einen Alkylrest darstellen, der gegebenenfalls mit einer Hydroxygruppe substituiert ist.

## Claims

1. Process for the synthesis of 2,2,2-trifluoroethanol by liquid phase catalytic hydrogenolysis of a compound of formula:

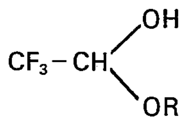

(I)

in which R is a hydrogen atom or a linear or branched alkyl radical containing from 1 to 8 carbon atoms and partially fluorinated if desired, in the presence of a nickel-based catalyst, characterized in that it is carried out in the presence of a cocatalyst chosen from aliphatic tertiary amines.

2. Process according to Claim 1, in which a mixture of a compound of formula (I) and of a cocatalyst is introduced progressively into a suspension of the catalyst in water or in 2,2,2-trifluoroethanol.

3. Process according to Claim 1 or 2, in which, after the reaction has ceased, the reaction mixture is rapidly cooled and the catalyst is separated off and is reused in a subsequent operation.

4. Process according to Claim 3, in which the catalyst is separated off by filtration and is then washed with water before reuse.

5. Process according to Claim 3, in which, after cooling of the reaction mixture, the catalyst is allowed to settle and then the reaction liquid is drawn off and the catalyst kept in the reactor is reused as such.

6. Process according to one of Claims 1 to 5, in which a compound of formula (I) is employed in the crude state.

7. Process according to one of Claims 1 to 6, in which the operation is performed at a temperature ranging from 150 to 200°C, preferably between 170 and 180°C.

8. Process according to one of Claims 1 to 7, in which the operation is carried out at a pressure of approximately 20 to 50 bars, preferably between 30 and 45 bars.

9. Process according to one of Claims 1 to 8, in which the nickel content of the catalyst varies from 30 to 90% by weight and is, preferably, equal to approximately 64%.

10. Process according to one of Claims 1 to 9, in which the catalyst is activated beforehand at a temperature between 140 and 200°C at a hydrogen pressure of 30 to 45 bars.

11. Process according to one of Claims 1 to 10, in which the quantity of catalyst, expressed for a catalyst containing 64% of nickel, is between 0.2 and 7.5%, preferably between 0.5 and 2%, relative to the weight of hydrate or hemiacetal employed.

12. Process according to one of Claims 1 to 11, in which the quantity of cocatalyst is between approximately 1.05 and 6 moles, preferably between 1.05 and 4 moles, per gram-atom of chlorine in the chlorinated byproducts present in the crude compound of formula (I).

13. Process according to Claim 12, in which the cocatalyst is an amine of formula $R_1-N(R_2)R_3$ in which the symbols $R_1$, $R_2$ and $R_3$, which are identical or different, each denote an alkyl radical optionally substituted by a hydroxyl group.